# EUROPEAN PATENT APPLICATION

(11) **EP 1 676 524 A1**
(43) Date of publication of application: **05.07.2006**
(21) Application number: 04107007.9
(22) Date of filing: 28.12.2004
(51) Int. Cl.: A61B 5/00

(54) **Arrangement for monitoring of a patient**

(71) Applicant: INSTRUMENTARIUM CORPORATION, 00510 Helsinki (FI)
(72) Inventor: Anttila, Mika, 02200, Espoo (FI); Niklander, Tapani, 02130, Espoo (FI)
(74) Representative: Valkeiskangas, Tapio Lassi Paavali

(57) **Abstract**

The invention relates to an arrangement for monitoring of a patient comprising one or several sensors for patient connection, a patient transceiver device and a transfer system, which is capable to transfer data between the sensors and the patient transceiver device, the arrangement further comprising a base station/monitor transceiver device at least to receive data sent by the patient transceiver device, and a battery device to act as a power source for the arrangement. The battery device (11) is attached into one of the sensors (9).

## Description

The invention relates to an arrangement for monitoring of a patient comprising one or several sensors for patient connection, a patient transceiver device and a transfer system, which is capable to transfer data between the sensors and the patient transceiver device, the arrangement further comprising a base station/monitor transceiver device at least to receive data sent by the patient transceiver device, and a battery device to act as a power source for the arrangement.

Monitoring of a patient provides basic diagnostic and clinical data in health care, such as blood pressure, pulse rate, biopotential signals, i.e. electro cardiograms (ECG), electroencephalograms (ECG), and electromylograms (EMG)/Entropy, and further temperature, blood oxygenation (Sp02), gas measurements, etc. Wireless monitoring devices, such as patient telemetry, have been developed to improve patient mobility and comfort. Wireless systems can also improve workflow efficiency of hospitalized patient by reducing the number of cables in the care environment.

The sensors acquiring physiological signals, such as ECG electrodes, Sp02 probes or temperature probes, can be reusable or disposable items. In many cases disposable items are preferred for hygienic and practical reasons.

Typical patient telemetry set up comprises four basic elements, namely 1. telemetry transmitter, for example ECG telemetry transmitter, 2. base station/receiver of the system, 3. electrodes, for example disposable ECG electrodes for patient connection, and 4. cable system connecting electrodes to the telemetry transmitter, for example ECG leadwires. The LifeSync® Wireless ECG System, and the system described in PCT document WO 2004/028344 can be mentioned as examples of the prior art solutions.

A typical patient telemetry transmitter has 2-3 AA(A) size battery cells acting as power source for the transmitter. It is a customer requirement for a wireless transmitter to operate long enough without battery re-charge/replacement. In many healthcare applications, especially in hospital care processes, continuous operation with one patient is required. Device recharge should be performed between patients. Examples of such processes would be patient transport, surgical operation or emergency admittance.

Although the capacity of the battery can last for more than one patient the recharge should be synchronized between patients to avoid any patient monitoring disruption. Various alarming and indicator systems have been developed to predict and indicate exhausting battery. These warning systems, like commonly used light emitting diodes (LED) or sound generators, consume also some of the remaining battery capacity expediting the exhaust process.

Despite of the battery technology development the size and the weight of the batteries limit the use of wireless systems. Battery replacement or recharge process needs to be managed. The device is out of use when re charged unless duplicate battery units are being purchased and circulated. Any disruption in the battery management can cause the system be out of operation when needed. Unexpected outages are highly intolerable in patient care. In other words the disadvantages of the prior art is uncertain battery management, i.e. one cannot be quite sure whether or not the batteries used will remain active as long as needed. It is quite possible always at the beginning of the monitoring step to replace the existing batteries by fresh ones but it is still quite possible that because a of human error said replacement step can be forgotten, and therefore human behaviour may rise to a dominating factor and may lead to difficult disadvantages in spite of modern technology used.

The object of the invention is to obtain an arrangement by means of which the disadvantages of the prior art can be eliminated. This is achieved with the invention. The arrangement of the invention is characterized in that the battery device is connected into one of the sensors.

The advantage of the invention is the battery replacement is a seamless operation for the user and the risk of not having batteries recharged when starting wireless monitoring is avoided. This is due to the fact that in the invention batteries can for example be embedded in a disposable part of the measuring system, i.e. in ECG electrodes. It is a normal clinical practice, and a part of a standard process to select and use fresh electrodes or similar disposable items for each patient. This is true for many physiological measurement systems and related disposable items. The batteries can be selected so that their capacity is well above what is needed for monitoring one patient in the intended care process application. Preferably non-toxic environmental friendly battery technology, like zinc air cells used in hearing aids, will be used to allow easy disposal of the single use item. The battery can be disposed with the electrode, when removed for full benefit of the invention, or it can be circulated for reuse if that is preferred. The invention offers improved workflow efficiency though simplified process and increased reliability for the measurement. This is due to the fact that new unused batteries are always automatically selected in connection with the use of normal clinical practice according to which fresh electrodes or similar are selected for each patient.

In the following the invention will be described in greater detail with reference to the accompanying drawings, in which
Figure 1 shows schematically a typical prior art wireless patient monitoring system,
Figure 2 shows one embodiment of the present invention,
Figure 3 shows a second embodiment of the present invention,
Figure 4 shows a third embodiment of the present invention,
Figure 5 shows a fourth embodiment of the present invention, and
Figure 6 shows a fifth embodiment of the present invention.

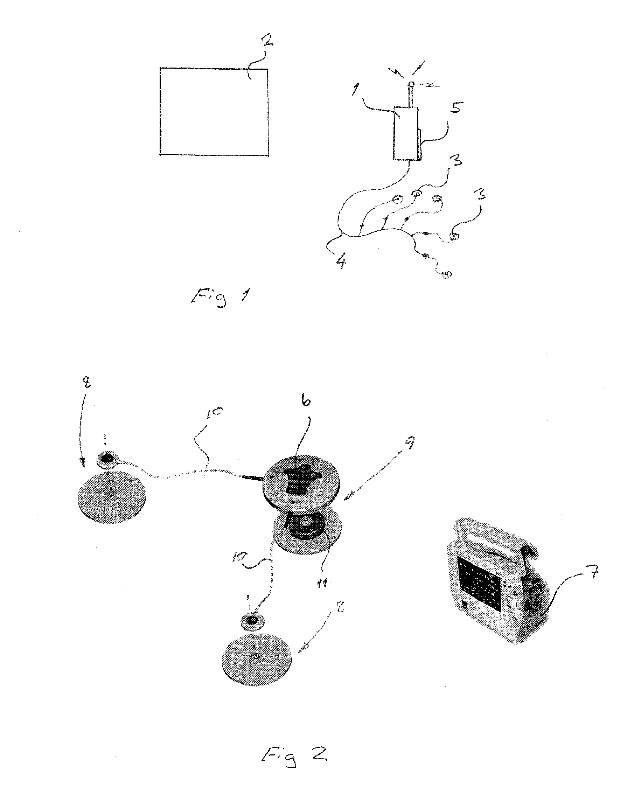

Figure 1 shows schematically a typical prior art wireless patient monitoring system. Reference number 1 shows an ECG telemetry transceiver device. Reference number 2 shows a base station/monitor transceiver device of the system. The base station/transceiver device receives the data wirelessly sent by the transceiver device 1. In this connection it must also be noted that the base station/monitor transceiver device can also send information to the transceiver device 1 if needed. Reference number 3 shows sensors for example disposable ECG electrodes for patient connection. Reference number 4 shows a transfer system, for example ECG leadwires connecting electrodes to the telemetry transmitter device 1. Reference number 5 shows a battery device that acts as a power source for the transmitter device 1. The battery device 5 can be for example a unit consisting 2-3 AA(A) size battery cells as described above, or for example a lithium battery unit.

The system shown in Figure 1 uses disposable ECG electrodes 3, i.e. fresh electrodes are selected for each patient. Other elements of the system shown in Figure 1 are reusable. The recharge or replacement of the battery device 5 must be synchronized between patients to avoid any patient monitoring disruption. The system shown in Figure 1 has the disadvantages of the prior art described above.

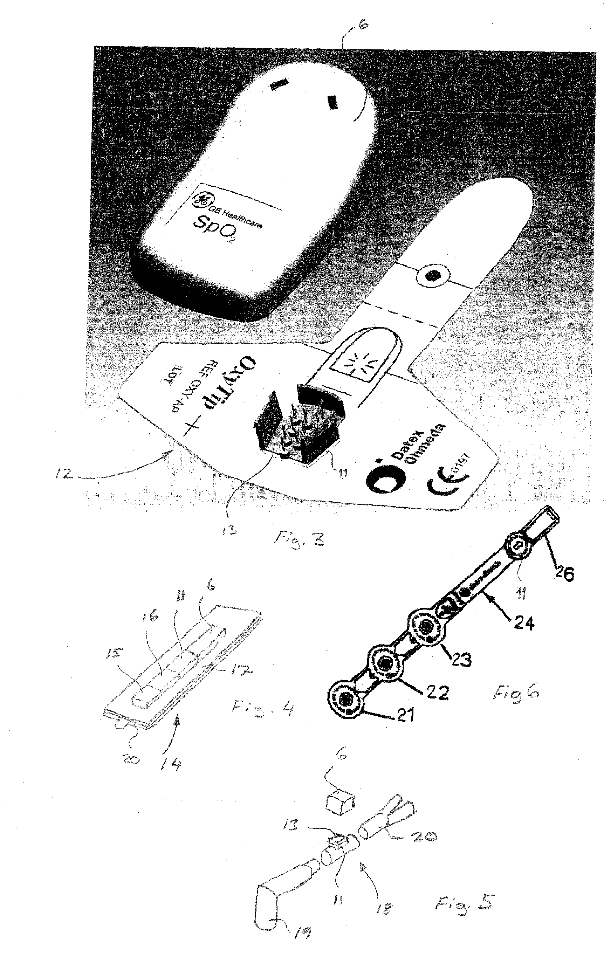

Figure 2 shows one embodiment of the invention. The embodiment shown in Figure 2 is a wireless ECG measurement system. The system shown in Figure 2 comprises the same elements as the system shown in Figure 1. Reference number 6 shows a patient transceiver device, reference number 7 shows a base station/monitor transceiver device, reference numbers 8 and 9 show the sensors, in this embodiment the electrodes for patient connection, reference number 10 shows a transfer system, in this embodiment a cable system, connecting the electrodes 8 and the patient transceiver device 6 and reference number 11 shows a battery device. Electrode 9 can be connected to the patient transceiver device 6 by using for example a snap-on connector. As told above reference number 8 and 9 show the electrodes, i.e. the disposable parts of the system. According to the basic idea of the invention the battery device 11 is attached into one of the disposable parts, i.e. in this embodiment to the electrode 9. When the user connects the patient transceiver device 6 to the electrode 9 the system is activated. It is also possible that the system is designed so that the system is transferred to energy saving mode if there is no signal to be measured.

As described above and shown in Figure 2 the battery device 11 is embedded in one ECG electrode 9. It is however within the spirit of the invention also to attach the battery device 11 to one electrode for example by using a cable part or some other connecting device. The battery device 11 can be placed into the electrode 9 during the manufacture process of the electrode 9. Other electrodes 8 can be standard electrodes without any battery device. All electrodes are single use disposable electrodes. In the embodiment shown the electrodes 8 are connected to the measurement electronics via lead wire cable or directly as shown by the electrode 9 in Figure 2. The electrode 9 with embedded battery device 11 has contact terminals for the physiological signal as well as for the power supply. When the monitoring is finished all electrodes 8, 9 will be disposed and fresh electrodes will be selected for the next patient. The system can be activated also by using other appropriate proceedings, for example by placing the battery device to a correct placement, by breaking an appropriate sealed switch, by removing an activating element such as an adhesive tape etc., or by using some other activating element.

Figure 3 shows a second embodiment of the invention. This embodiment is based on a disposable Sp02 probe 12 which has light emitting diodes, light detector and related electronics built on a adhesive tape structure. These probes are commonly used in clinical practice. The sensor 12 is disposed after use. In this embodiment the battery device 11 is embedded to the Sp02 sensor design. In Figure 3 reference number 13 shows an electrical connector to which a cable or the patient transceiver device 6 can be connected. The battery device 11 can be placed for example under the electrical connector 13. When the monitoring electronics is connected to the sensor, it gets powered through the sensor connection and is able to acquire and send the clinical data to the base station/monitor transceiver device having a displaying unit. In this connection it must be understood that also other activating systems, e.g. those described above, can be used in the embodiment of Figure 3. The patient transceiver device 6 is preferably a rather versatile device, and therefore it is advantageous to use the patient transceiver device as a reusable device, i.e. the patient transceiver device is intended to be used with several measurements or patients, and therefore a reusable device is advantageous. It is however quite possible, at least in certain circumstances, to use a single use disposable patient transceiver device as well.

Figure 4 shows a third embodiment of the invention. This embodiment is a sensor 14, which consists of thermistor 15, temperature measurement electronics 16, battery device 11 and patient transceiver device 6 built on an adhesive tape structure 17. When said temperature measuring probe is placed on the skin of the patient the measurement is initiated. Also other activating principles can be used in this embodiment, for example adhesive tape 20, which can be removed in order to activate the system. In this embodiment the whole unit can be disposable and disposed after use. It is however naturally quite possible also to make an embodiment in which the patient transceiver device 6 is a separate non-disposable unit, which can be connected to the probe in the way as shown in Figure 3.

Figure 5 shows a fourth embodiment of the invention. This embod i-ment is a gas sensor, which can be connected for example to a duct in which respiratory gas from the patient flows. The respiratory gas to be measured flows through a duct 19 to a disposable gas sensor unit 18, which comprises a measuring chamber. According to the invention the gas sensor unit 18 comprises also the battery device 11. Reference number 20 shows a Y-piece, which can be connected to the gas circulating system of a ventilator. In Figure 5 reference number 6 shows a patient transceiver device, which can be connected to the gas sensor unit 18 basically in the same way as shown in Figure 3 by using an electrical connector 13.

Figure 6 shows a fifth embodiment of the invention. This embodiment is an EEG or EMG sensor, which can be used for measuring the depth of anesthesia. In this sensor three EEG or alternatively EMG electrodes 21, 22 and 23 are parts of a disposable sensor structure 24. The electrodes provide a skin contact during the use of the sensor. The battery device 11 is attached to the disposable sensor structure 24. The reference number 26 shows a non-disposable transceiver connected to the sensor.

The embodiments of the invention described above are by no means intended to restrict the invention, but the invention can be modified freely within the scope of the claims.

## Claims

1. Arrangement for monitoring of a patient comprising one or several sensors for patient connection, a patient transceiver device and a transfer system, which is capable to transfer data between the sensors and the patient transceiver device, the arrangement further comprising a base station/monitor transceiver device at least to receive data sent by the patient transceiver device, and a battery device to act as a power source for the arrangement, **char acterized in that** the battery device (11) is attached into one of the sensors (9, 12, 14, 18, 24).

2. The arrangement as claimed in claim 1, **characterized in that** the battery device (11) and the sensors (9, 12, 14, 18) are single use disposable devices.

3. The arrangement as claimed in claim 1, **characterized in that** the patient transceiver device is a non-disposable device.

4. The arrangement as claimed in claim 1, 2 or 3, **characteriz ed in that** the battery device (11) is provided with an activating element (20), which can be removed or broken to activate the arrangement.

5. The arrangement as claimed in any of the preceding claims 1 to 4, **characterized in that** the sensors comprise biopotential electrodes

6. The arrangement as claimed in claim 5, **characterized in that** the sensors (9) comprise ECG electrodes.

7. The arrangement as claimed in claim 5, **characterized in that** the sensors (21, 22, 23) comprise EEG electrodes.

8. The arrangement as claimed in claim 5, **charactrized in that** the sensors are (21, 22, 23) comprise EMG electrodes.

9. The arrangement as claimed in any of the preceding claims 1 to 4, **characterized in that** the sensors (12) comprise Sp02 probes.

10. The arrangement as claimed in any of the preceding claims 1 to 4, **characterized in that** the sensors (17) comprise temperature measuring probes.

11. The arrangement as claimed in any of the preceding claims 1 to 4, **characterized in that** the sensors (18) comprise gas sensor units.

12. The arrangement as claimed in claim 1 or 2, **characterize d in that** the patient transceiver device (6) is placed into one of the sensors (9, 12,14,18).

13. The arrangement as claimed in claim 12, **characterized in that** the patient transceiver device (6) is a single use disposable device.

14. The arrangement as claimed in claim 1, **characterized in that** the data between the patient transceiver device and the base station/monitor transceiver is arranged to be wirelessly sent.

15. The arrangement as claimed in claim 1, **characterized in that** the battery device (11) is placed into one of the sensors (9, 12, 14, 18, 24).
